# EUROPEAN PATENT SPECIFICATION

(11) **EP 3 160 993 B1**
(45) Date of publication and mention of the grant of the patent: **27.06.2018**
(21) Application number: 15745135.2
(22) Date of filing: 22.06.2015
(51) Int. Cl.: C07K 14/62

(54) **INSULIN DERIVATIVE WITH CYCLIC STRUCTURE IN THE C-TERMINUS OF THE B-CHAIN**
INSULINDERIVAT MIT ZYKLISCHER STRUKTUR IN C-TERMINUS DER B-KETTE
DÉRIVÉ D'INSULINE AVEC STRUCTURE CYCLIQUE DANS LE C-TERMINALE DE LA CHAÎNE B

(30) Priority: 30.06.2014 CZ 20140450
(43) Date of publication of application: 03.05.2017
(73) Proprietor: Ustav Organicke Chemie A Biochemie Akademie Ved Cr, v.v.i., 16610 Praha (CZ); The University of York, York, Yorkshire YO10 5DD (GB)
(72) Inventor: JIRACEK, Jiri, 25091 Svemyslice (CZ); ZAKOVA, Lenka, 14000 Praha 4 (CZ); VANEK, Vaclav, 14100 Praha 4 (CZ); VEVERKA, Vaclav, 17000 Praha 7 (CZ); BRZOZOWSKI, Andrzej Marek, York Yorkshire YO10 5DD (GB)
(74) Representative: Hartvichova, Katerina
(86) International application number: PCT/CZ2015/000065
(87) International publication number: WO 2016/000667

(56) References cited:
- WO-A2-2014/071405
- TINE GLENDORF ET AL: "Engineering of Insulin Receptor Isoform-Selective Insulin Analogues", PLOS ONE, vol. 6, no. 5, 20 May 2011 (2011-05-20), page e20288, XP055216887, DOI: 10.1371/journal.pone.0020288 cited in the application
- JIRACEK J ET AL: "Novel insulin analogues with cyclic structures mimicking the active conformation of the hormone at the C-terminus of the B-chain", JOURNAL OF PEPTIDE SCIENCE, JOHN WILEY AND SONS LTD, GB, vol. 18, no. Suppl.1, 1 September 2012 (2012-09-01), page S41, XP009186354, ISSN: 1075-2617
- J. JIRACEK ET AL: "Implications for the active form of human insulin based on the structural convergence of highly active hormone analogues", PROCEEDINGS OF THE NATIONAL ACADEMY OF SCIENCES, vol. 107, no. 5, 2 February 2010 (2010-02-02), pages 1966-1970, XP055217232, ISSN: 0027-8424, DOI: 10.1073/pnas.0911785107 cited in the application
- KURTZHALS P ET AL: "CORRELATIONS OF RECEPTOR BINDING AND METABOLIC AND MITOGENIC POTENCIES OF INSULIN ANALOGS DESIGNED FOR CLINICAL USE", DIABETES, AMERICAN DIABETES ASSOCIATION, US, vol. 49, no. 6, 1 June 2000 (2000-06-01), pages 999-1005, XP002902916, ISSN: 0012-1797, DOI: 10.2337/DIABETES.49.6.999

## Description

### Field of the Invention

The invention relates to a new insulin derivative with high binding affinity to both isoforms of the insulin receptor. Affinity to isoform B is significantly higher than affinity to isoform A; the derivative also exhibits a very low affinity to the IGF-1 receptor.

### Background Art

The metabolic disease diabetes mellitus affects millions of people worldwide. Statistical estimations speak clearly: the number of people affected by diabetes will continue to increase and there will be approximately 550 million diabetics by 2030. This is why research in the field of insulin interaction with the insulin receptor is very important, as it could lead to the development of new insulin derivatives with features that increase the quality of life of diabetic patients.

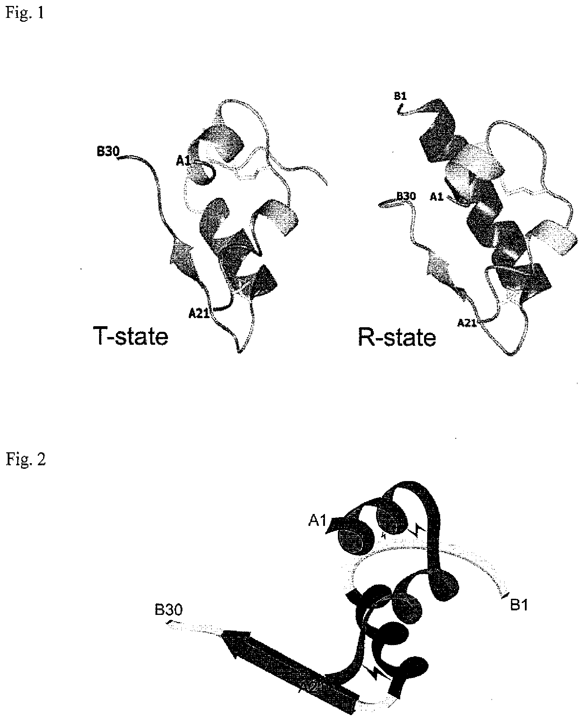

Insulin is a peptide hormone and its main role is the regulation of blood glucose levels and facilitation of its entry into cells. Besides that, insulin has a major impact on protein and fat metabolism. Insulin's primary structure consists of peptide chains A (21 aminoacids) and B (30 aminoacids), which are connected via two intermolecular disulfide bridges (A7-B7 a A20-B19). Then there is one intramolecular bridge in the A-chain (A6-A11). The secondary and tertiary structure of insulin are shown schematically in Fig 1.

The A-chain forms two helices. The B-chain only forms a single helix in its middle section in the T-state. In the R-state (which is only formed in the presence of small cyclic alcohols such as *e.g.* phenol), this helix in the B-chain continues to its N-terminal aminoacid B1. The physiological role of the T- and R-conformations is not yet known.

The biological effect of insulin is primarily mediated by the insulin monomer binding to the insulin receptor (IR). The insulin receptor is a tetrameric membrane glycoprotein consisting of two extracellular α-subunits and two transmembrane and intracellular β-subunits. The subunits are connected via several disulfide bridges (McKern N. M., Lawrence M. C. et al., Nature 2006, 443, 218). Insulin binding to the receptor activates a tyrosine kinase, which is part of the intracellular part of the β-subunit, and then a signal cascade is activated, triggering the biological effects of insulin.

There are two isoforms of the insulin receptor due to alternative splicing of exon 11 (aminoacids 717-729); IR-A (- exon 11) and IR-B (+ exon 11). The difference between them is a sequence of 12 aminoacids that is located at the C-terminus of the receptor α-subunit and is called the αCT-peptide. The αCT-peptide is very important for the receptor's binding properties and its tissue distribution. IR-B is the "classical" form of the receptor that binds insulin alone under physiological concentrations. IR-B is primarily expressed in the liver, muscle tissue and fat tissue. The relative abundance of IR-B versus IR-A is ∼100 % in adult liver, 80 % in fat tissue and 50-70 % in muscles (Moller D. E., Yokota A. et al., Mol. Endocrinol 1989, 3, 1263). IR-B mainly triggers the metabolic signalling pathway ending with glucose entry into the cell. IR-A is mainly expressed at prenatal age; at adult age mainly in the brain, spleen, and also in cancer cells (De Meyts P., Endocrinology 2012, 153,2054).

Both isoforms (IR-A and IR-B) bind insulin with similar affinity (0.2-0.3 nmol.l⁻¹). In contrast to IR-B, IR-A (besides insulin) also binds the hormone IGF-2 in nanomolar concentrations, and also IGF-1, although somewhat more weakly (Insulin-like growth factors 1 and 2) (Frasca F., Pandini G. et al., Mol. Cell. Biol. 1999, 19, 3278; Morcavallo A., Genua M. et al., J. Biol. Chem. 2012, 287, 11422). The activation of IR-A by IGF hormones leads primarily to mitogenic effects (Frasca F., Pandini G. et al., Mol. Cell. Biol. 1999, 19, 3278). The complexity of the system is highlighted by the fact that both IR isoforms can form heterodimers with each other or with the subunits of the IGF-1 receptor (IGF-1R). The IGF-1R:IR-A hybrid is for example only a receptor for IGF hormones and doesn't bind insulin (Malaguarnera R., Belfiore A., Front. Endocrinol. 2012, 2,1).

Insulin binds to extracellular α subunits, but the complex structure of insulin bound to the receptor is not precisely known yet.

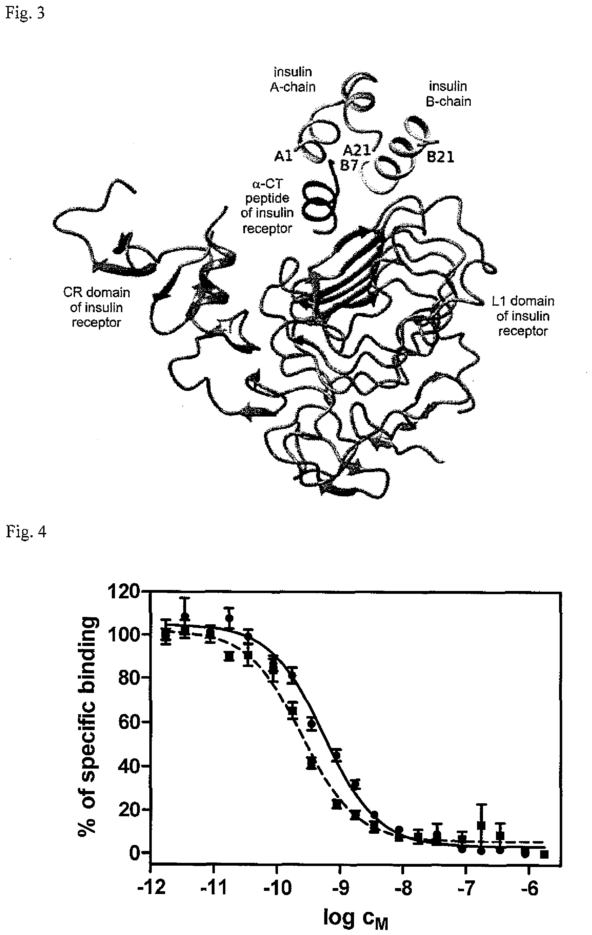

The recent solution of the crystal structure of the insulin complex with insulin receptor constructs (Menting J. G., Whittaker J. et al., Nature 2013, 493, 241) was an important milestone in research into insulin's interaction with its receptor. Fig 3 shows the interaction of insulin with the L1 domain of the receptor and the αCT peptide (from the A isoform). This structure does not provide any information about the second binding site of insulin and receptor (FnIII domain), nor either of the flexible parts of insulin, i.e. the N- and C-termini of the B-chain (they are not visible). However, the visible interactions of the central part of B-chain with the αCT peptide and L1 domain and the interaction of A-chain with the αCT peptide confirmed the above-mentioned assumptions about the insulin - receptor interaction. Another interesting and important finding was that if the C-terminus of the B-chain was in the same conformation as in the storage forms of insulin (Fig. 1), it would collide with the αCT peptide located at the L1 domain. The structure in Fig. 3 shows that at least part of the insulin B-chain must be deflected from the insulin central helix similarly as indicated in Fig. 2. Fig 2 also shows that even the bent C-terminus of the B-chain can most probably interact with αCT; probably differently for αCT peptides from the A and B isoforms.

Derivatives or analogues of insulin are compounds derived in their structure from the structure of the human insulin molecule, but targeted modifications in the aminoacid sequence of the A- or B-chain result in their amended biological effects and a different binding affinity to the receptor.

Derivatives of insulin are developed particularly for improving the quality of diabetes treatment and making the patients more comfortable. Another important reason for preparing the derivatives is work to clarify the interaction of insulin with the insulin receptor, because as mentioned above, the interaction of these two has not yet been explored in detail and the crystal complex of both molecules has not been prepared yet.

There are currently 6 pharmaceutical compositions on the market and used in medical practice containing these derivatives. Three of them are rapid-acting insulin derivatives (Lispro, Aspart a Glulisine). They are characterized by a faster onset and faster subsidence of their effects compared to human insulin. The other three derivatives (Glargine, Detemir a Degludec) are long-acting derivatives and are characterized by a duration of action that is several times longer. The principle of the action of these clinically used derivatives is a change in their solubility compared to natural insulin. This is primarily achieved by influencing their ability to dimerize and form hexamers, changing the isoelectric point (pI) of derivative molecules or increasing their ability to bind to serum proteins.

Some of the clinically used insulin analogues exhibit a sequence similarity to IGF-1 and 2, they have higher affinity to IGF-1R than human insulin and they also exhibit higher mitogenic activity than human insulin. Currently, the relationship between the use of these analogues and cancer prevalence is intensively studied. The potential risks are mainly associated with the long-acting analogues, such as Glargine (Kurtzhals P., Schaffer L. et al., Diabetes 2000, 49, 999).

Insulin resistance and hyperinsulinemia may also increase the risk of cancer due to the activation and deregulation of the expression of IR, in particular the IR-A isoform (Belfiore A., Frasca F. et al., Endocr. Rev. 2009, 30, 586). The ability of IGF-2 to bind to IR-A plays an important role in this mechanism. Recently, the term IGF-2-oma was introduced for tumors that secrete IGF-2 (Dynkevich Y., Rother K. I. et al., Endocr. Rev. 2013, 34, 798). The binding of IGF-2 to IR-A, IGF-1R and the hybrid receptor IR-A/IGF-1R subsequently causes an increased expression and activation of these receptors and IGF-2, resulting in the formation of a growth-promoting loop IR-A/IGF-1R/IGF-2. Insulin analogues with enhanced binding selectivity in favour of IR-B and a reduced affinity for IGF-1R would thus be highly desirable.

### Disclosure of the Invention

This invention describes a new derivative of human insulin, said derivative corresponding to formula I, having a cyclic structure in the C-terminus of the B-chain. The derivative **I** exhibits a higher affinity to both isoforms of the insulin receptor than that of human insulin, and at the same time it has a markedly higher affinity to the predominantly metabolic B isoform than to the predominantly mitogenic A isoform of this receptor. Another advantage is a significantly lower affinity of the derivative to IGF-1R than that of human insulin. These properties predetermine the new insulin derivative of formula **I** for use as effectively acting insulin, lowering blood glucose levels *in vivo,* with lower side effects, in particular a lower possibility of developing cancer, than those of human insulin.

The object of this invention is the insulin derivative of formula **I**, 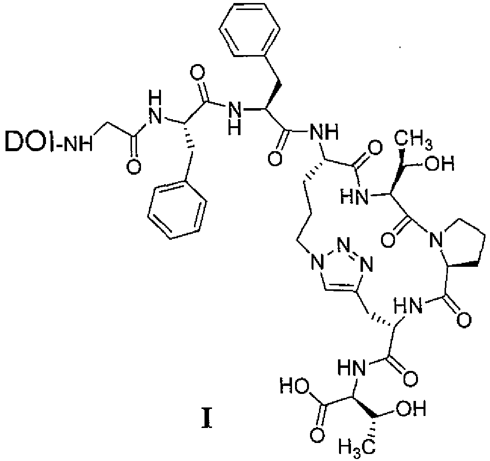 where **DOI** is des(B23-B30)octapeptide-insulin, and its pharmaceutically acceptable salts and solvates.

**DOI,** or des(B23-B30)octapeptide-insulin, is human insulin without the C-terminal octapeptide B23-B30. In the derivative of formula **I, DOI** is bound to modified octapeptide of formula **II** simulating the sequence B23-B30 of human insulin via a peptide bond between the C-terminal carboxyl group of **DOI** in the B22 position and the amino group of the glycine at the N-terminus of the modified octapeptide of formula **II**

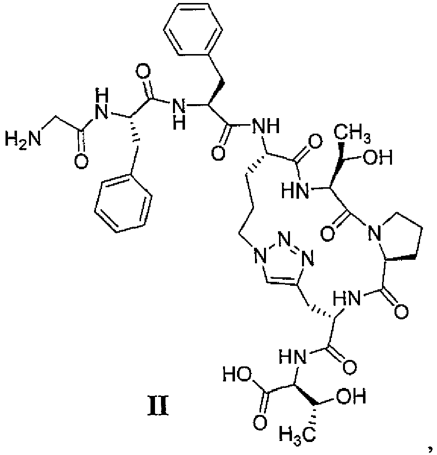

Another aspect of the invention is also the insulin derivative of formula I according to the invention, or its pharmaceutically acceptable salts or solvates, for use as a medicament.

The aspect of the invention is also the insulin derivative of formula **I** according to the invention, or its pharmaceutically acceptable salt or solvate, for use for the treatment or prevention of a condition involving hyperglycemia (elevated blood sugar level) or for diabetes treatment.

Another object of this invention is also the use of the insulin derivative of formula **I** or its pharmaceutically acceptable salt or solvate, in the manufacture of a medicament for the treatment or prevention a condition involving hyperglycemia (elevated blood sugar level) or for diabetes treatment.

The object of the invention is also a pharmaceutical composition comprising the insulin derivative of formula **I** or its pharmaceutically acceptable salts or solvates, or mixtures thereof.

The pharmaceutical composition of the present invention may further contain at least one pharmaceutically acceptable carrier, excipient and/or diluent.

The invention further includes the pharmaceutical composition for use for the treatment or prevention of a condition involving hyperglycemia (elevated blood sugar level) or for diabetes treatment.

The term "pharmaceutically acceptable salts" refers to salts of cations or anions covered by formula I with counter ions that are acceptable for pharmaceutical use. Examples of pharmaceutically acceptable salts are salts of alkali metals and alkaline earth metals, ammonia salts, metal salts, salts of anions of inorganic or organic acids, such as halides, sulphates, carbonates, acetates, succinates, etc.

The solvates contain one or more molecules of the compound of formula **I** and one or more molecules of solvent which is acceptable for use in pharmaceutical compositions, such as water.

The derivative of formula **I** according to this invention exhibits two-fold higher binding affinity for the insulin receptor (IR-A isoform) in the membranes of human IM-9 lymphocytes and particularly five-fold higher binding affinity for the insulin receptor in the membranes of murine fibroblasts transfected with the B isoform (IR-B) of the human insulin receptor that that of human insulin. Hence, the derivative is 2-5-fold more active more towards insulin receptor than human insulin and moreover with a significant preference for the metabolic isoform B of IR. Besides this, the derivative of formula **I** displays 5-fold lower binding affinity than human insulin against the IGF-1 receptor (IGF-1R)) in the membranes of mouse fibroblasts transfected with human IGF-1R.

Thus, the novel insulin derivative of formula **I** combines in its properties not only the preferential affinity for IR-B, which is the metabolic isoform of insulin receptor, but also very high binding affinity generally (515% compared to human insulin) and, hence, the derivative of formula **I** belongs to the most potent insulin derivatives ever. Especially, the low affinity of the derivative of formula **I** towards IGF-1 receptor is unique. Similar properties have not been described yet for any other insulin derivative and predetermine the derivative of formula **I** for the clinical use to effectively lower the blood glucose levels in diabetics with a lower risk of the development of cancer (due to the low binding to IGF-1R). The almost 5-fold higher binding affinity than that of human insulin for IR-B isoform may also enable the use of lower doses of this derivative than of human insulin, which could result in a cheaper treatment.

### Brief Description of the Drawings

Fig. 1 shows schematically the structures of the insulin monomer in the T-state (left) and in the R-state (right).
Fig. 2 shows the predicted deflection of the C-terminus of the B-chain of insulin (dark grey arrow) from the central part of the molecule.
Fig. 3 shows the structure of the complex of insulin with the insulin receptor. Insulin receptor is represented by L1 domain, CR-domain and αCT peptide. The N-terminal (A1 and B7) and the C-terminal (A21 and B21) insulin residues are marked by labels.
Fig. 4 shows the binding curves of human insulin (●) and insulin derivative I (■) to the insulin receptor (IR-A isoform) in the membranes of human IM-9 lymphocytes. The x-axis represents the log of the molar concentration of the tested insulin and derivative; the y-axis plots the degree of binding of radioactively labelled ¹²⁵I-insulin (in %), which is displaced from the receptor binding by unlabelled insulin or its derivative.

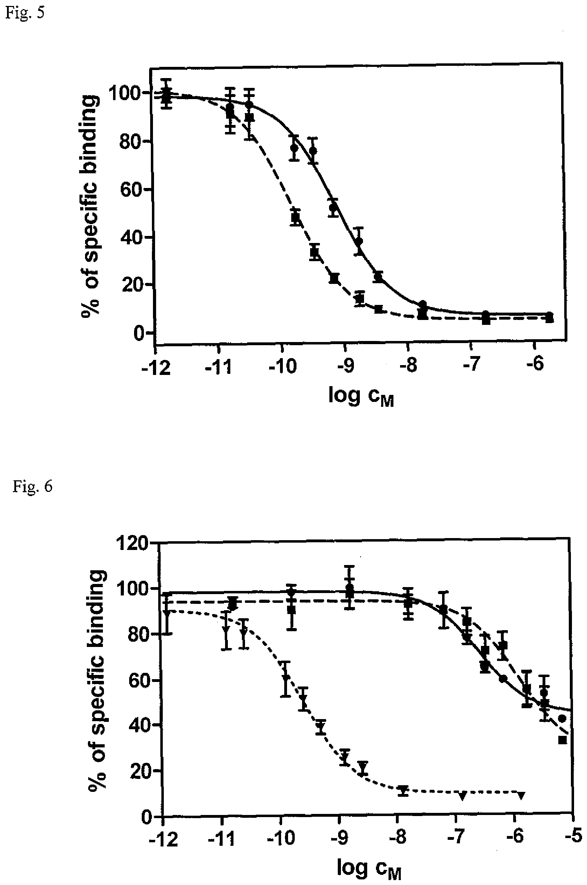

Fig. 5 shows the binding curves of human insulin (●) and insulin derivative I (■) to the insulin receptor (IR-B isoform) in the membranes of murine fibroblasts. The x-axis represents the log of the molar concentration of the tested insulin and derivative; the y-axis plots the degree of binding of radioactively labelled ¹²⁵I-insulin (in %), which is displaced from the receptor binding by unlabelled insulin or its derivative.
Fig 6 shows the binding curves of human insulin (●), insulin derivative I (■) and human IGF-1 (▼) to the IGF-1R receptor in the membranes of murine fibroblasts. The x-axis represents the log of the molar concentration of the tested insulin and derivative; the y-axis plots the degree of binding of radioactively labelled ¹²⁵I-IGF-1 (in %), which is displaced from the receptor binding by unlabelled insulin, IGF-1 or derivative **I.**

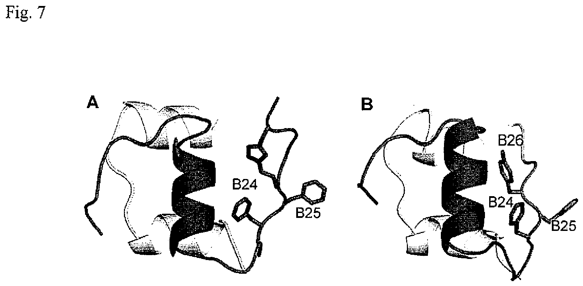

Fig. 7 shows a comparison of the NMR structures of a representative insulin derivative **I** (A) and human insulin (PDB code 2HIU) (**B**) measured in an environment of 20% acetic acid. A-chains are indicated by light grey and B-chains by dark grey. For clarity, the side chains are also displayed (PheB24, PheB25, TyrB26 in human insulin and PheB24, PheB25 and the triazole bridge in the derivative **I**.)

### Examples

### List of abbreviations:

Bz benzyl
DIPEA N,N-diisopropylethylamine
DCM dichloromethane
DIC N,N-dicyclobexylcarbodiimide
DOI des(B23-B30)octapeptide-insulin
DMF N,N-dimethylformamide
Fmoc 9-fluorenylmethyloxycarbonyl
HBTU 2-(1H-benzotriazole-1-yl)-1,1,3,3-tetramethyluronium hexafluorophosphate
HOBT N-hydroxybenzotriazole
IR insulin receptor
IGF insulin-like growth factor
   IGF-1Rreceptor for insulin-like growth factor
NMP N-methyl-2-pyrrolidone
TFE 2,2,2-trifluoroethanol
RP-HPLC reverse phase high-performance liquid chromatography
HR-ESI high-resolution electrospray ionization mass spectrometry
TPCK p-tosyl-L-fenylalaninchloromethylketone
   HEPES 2-[4-(2-hydroxyethyl)piperazin-1-yl]ethanesulfonic acid
EDTA ethylenediaminetetraacetic acid
BSA bovine serum albumin
MeOH methanol
t-BuOH t-butanol

### A) Preparation of the derivative of formula I

### Preparation of linear precursor III for cyclic octapeptide II

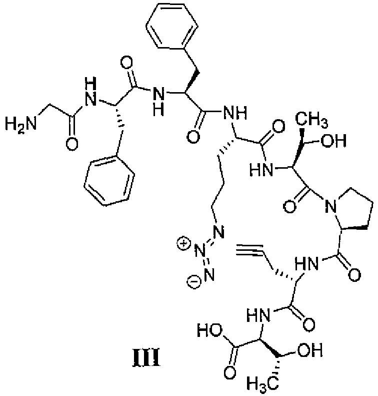

^{T}he linear octapeptide III was prepared by solid phase synthesis. Highly acid-labile 2-Cl-trityl resin (Merck-Novabiochem) was used as the polymeric carrier (usually in an amount corresponding to 400 x 10⁻⁶ mol of active groups). Manual synthesis was carried out in a plastic syringe with a frit. For the synthesis of the octapeptide, amino acids were used with primary α-amino groups protected using Fmoc and the protected hydroxyl groups of the side chains using tertiary butyl (tBu). (S)-5-Azido-2-(Fmoc-amino)pentanoic acid (Sigma-Aldrich, cat. No. 714291) was placed at the B26 position in the linear octapeptide and (S)-2-(Fmoc-amino)-4-pentynoic acid (Sigma-Aldrich, cat. No. 00397) at position B29. Attaching the first amino acid to the resin was achieved by addition of the amino acid (1 eq) in DCM in a total volume of 1 ml. The reaction proceeded for about 2 hours at room temperature. The resin-bound amino acid was then left for approximately 2 x 10 minutes in 2 x 2 ml of DCM:MeOH:DIPEA (17:2:1). This led to deactivation and blocking of the remaining unreacted resin chloride groups. The protecting group (Fmoc) of the primary α-amino group was removed by repeated (5 and 20 min) treatment with 1 ml of a 30% (vol./vol.) solution of piperidine in DMF. The success of the reaction was evaluated spectrophotometrically by measuring the optical density of the resulting dibenzofulvene-piperidine complex at a wavelength of 301 nm (ε = 7040 M⁻¹cm⁻¹). The binding of the other amino acid was catalysed using a coupling agent HBTU and DIPEA in 2 ml of NMP. The reaction always proceeded for at least 1 hour. The ratio of the amounts of the individual reaction ingredients (amino acid: HBTU: DIPEA) was equal to 3:3:6 relative to the amino group present on the resin (1 equivalent). Alternatively, when the mixture was allowed to react overnight, the reagents HOBt and DIC were used in a solvent mixture of DCM and NMP in a 1:1 ratio (1 ml). In this case, the amounts of all reaction components were equimolar. Verifying the successful completion of the reaction was done using the Kaiser test (Kaiser E., Colescott R. L. et al., Anal. Biochem. 1970, 34, 595) for the presence of primary amino groups. According to the test result, either the reaction was repeated with the same aminoacid, or it was followed by removal of the protecting Fmoc group of the final bound aminoacid. The octapeptide was cleaved from the polymer resin carrier by treatment with 5 ml of 20% (vol. / vol.) TFE and 20% (vol. / vol.) acetic acid in DCM for 2 hours. The cleavage product was then evaporated to dryness and the remaining protecting groups (tBu) were removed by treatment with 5 ml of 50% trifluoroacetic acid (TFA), 2% triisopropylsilane and 2% H₂O in DCM for 1 hour; all percentages are percent by volume. The product was then evaporated to dryness again, washed three times with diethyl ether and dissolved in 10% acetic acid. Octapeptide was further purified by RP-HPLC, lyophilized and identified by mass spectrometry. Purity by RP-HPLC was greater than 95%. The yield after purification by RP-HPLC was 55% (143 mg, 84 µmol) and is calculated with respect to the substitution of the resin obtained after the first condensation of the first amino acid (285 µmol, tj. 100%). **HR-ESI** C₄₃H₅₇N₁₁O₁₁ [M+H]⁺ calculated 904.432, found 904.432.

### Preparation of cyclic octapeptide II

The linear octapeptide **III** (46 mg, 51 µmol, 1 molar equivalent) was dissolved in 650 ml of bled (vacuum, Ar) mixture of water : tBuOH (2:1). Ascorbic acid (15 equivalents), CuSO₄.5H₂O (10 equivalents) and tris[(1-benzyl-1H-1,2,3-triazol-4-yl)methyl]amine (TBTA, 6 equivalents) in 100 ml of the same mixture of water : tBuOH (2:1) were added to the solution. After brief stirring, the reaction mixture was allowed to stand at room temperature in the dark. The reaction was monitored by RP-HPLC before and after addition of the reagents. Formation of the cyclic product was observed as the disappearance of the starting linear peptide and the formation of a new product with a shorter retention time. The reaction mixture was evaporated on a rotary evaporator to a volume of about 10 ml and the peptide was desalted in a reverse-phase carrier column (Chromabond® C-18, volume cca. 12 ml). The desalted product was further purified with RP-HPLC, lyophilized and identified by mass spectrometry. Purity by RP-HPLC was greater than 95 %. The yield after RP-HPLC purification was 58 % (27 mg, 30 µmol). **HR-ESI** C₄₃H₅₇N₁₁O₁₁ [M+H]⁺ counted 904.432, found 904.432.

### Preparation of insulin derivative I

A new insulin derivative of formula **I** was prepared by the method of enzymatic semi-synthesis according to Žáková *et al.* (Žáková L., Zyka D. et al., J. Pept. Sci. 2007, 13, 334). The cyclic octapeptide (18.8 mg, 21 µmol) and DOI (29.2 mg, 5.4 µmol) were dissolved in a mixture (total volume 200 µl) containing 55% (vol./vol.) DMF in water, 20 x 10⁻³ mol.l⁻¹ calcium acetate and 1.9 mg TPCK trypsin (Sigma-Aldrich, T-1426 or T-8802). The pH of the solution was adjusted to 6.9-7.0 by the addition of 5 microliters of to the *N*-methylmorpholine. The reaction mixture was allowed to react with stirring at room temperature for 24 hours. The formation of products was monitored by RP-HPLC. The reaction was stopped by adding chilled acetone. The precipitate was dissolved in 10% (vol./vol.) acetic acid in water and the product was purified by RP-HPLC. The identity of the product was confirmed by mass spectrometry in a LTQ Orbitrap XL from Thermo Fisher Scientific. The purity according to RP-HPLC was greater than 95%. The yield (relative to DOI as the limiting compound of the reaction) after RP-HPLC purification was 17 % (5.3 mg; 0.92 µmol). HR-ESI [M] counted 5747.58, found 5747.58.

### B) Binding affinity of insulin derivative of formula I to IRA isoform of human insulin receptor in membranes of IM-9 lymphocytes

The principle of testing the binding affinity of the insulin derivatives is the competition of the derivative with human insulin, radioactively labelled on tyrosine at position A14 using the isotope ¹²⁵I(Perkin-Elmer, USA, 2200 Ci/mmol, τ=60 days), for the binding site on the insulin receptor. The *in vitro* human lymphocyte cell line IM-9 with a high level of expression of the insulin receptor isoform A was used to test the binding affinities of the insulin derivative to insulin receptor isoform A (IR-A). Procedures by De Meyts (De Meyts P. 1976, Methods in Receptor Research, M. Dekker, New York, 301) and Morcavallo *et al.* (Morcavallo A., Genua M. et al., J. Biol. Chem. 2012, 287, 11422) were used for the testing.

IM-9 cells were grown according to the recommended procedure of the supplier (ATCC, Manassas, USA; LGC Standards, Poland). Cells were grown under a humidified atmosphere with 5% CO₂ at 37 °C in a commercially available RPMI-1640 medium containing 10% fetal bovine serum (wt./vol.), 2 x 10⁻³ mol.l⁻¹ glutamine and 100 U/ml penicillin/streptomycin (all chemicals from Invitrogen, Carlsbad, California, USA). To ensure optimal growth conditions, the cells were passaged 3 times per week. Cells were first counted and then diluted to a concentration of 2 million / ml.

Stock solutions of insulin and its derivative were prepared in 0.1% acetic acid. Concentrations were determined by measuring absorbance at 280 nm and extinction coefficients (ε), 5840 M⁻¹.cm⁻¹ for human insulin, or 4560 M⁻¹.cm⁻¹ for the derivative **I** with only three tyrosines. By diluting with binding buffer (see below), solutions with decreasing concentrations of the tested insulin derivative were subsequently prepared. The cells were incubated in the reaction mixture with a solution of the insulin derivative of the appropriate diluted concentration and a constant amount of radiolabeled insulin (20,000 cpm). The reaction proceeded for 2.5 h at 15 °C in a binding buffer with the following composition: 100 x 10⁻³ mol.l⁻¹ HEPES/NaOH pH 7,6; 100 x 10⁻³ mol.l⁻¹ NaCl, 5 x 10⁻³ mol.l⁻¹ KCl, 1,3 x 10⁻³ mol.l⁻¹ MgSO₄, 1 x 10⁻³ mol.l⁻¹ EDTA, 10 x 10⁻³ mol.l⁻¹ glucose, 15 x 10⁻³ mol.l⁻¹ sodium acetate a 1% BSA (wt./vol.). The total reaction volume was 500 µl. The mixture was stirred every 30 minutes. From each tube, duplicates of 200 µl were created and the reaction was stopped by adding 200 µl of binding buffer at 4 °C. The mixture was then centrifuged at 15000 g for 10 min at 4 °C. After aspiration of the supernatant, the radioactivity of the pellet was measured in the γ-computer. The data were analysed using a model of binding to one binding site with a programme created in Excel and developed in the laboratory of Pierre De Meyts in Denmark (Groth A. V., Shymko R. M., Hagedorn Research Institute, Dánsko, 2008, dar P. de Meytse), or with GraphPad Prism 5.0 using the same model of binding to one site by non-linear regression, taking into account the potential depletion of the ligand. The value of the dissociation constant (*K*_{d}) of the derivative or of human insulin to the receptor was determined. The concentration of ¹²⁵I-radiolabeled insulin was 0.01 x 10⁻⁹ mol.l⁻¹. A value of 0.3 x 10⁻⁹ mol.l⁻¹ was used as the *K*_{d} of the ¹²⁵I-labelled insulin to the receptor.

The results of the binding studies with insulin derivative **I** and human insulin are shown in Table 1 and in Figure 4.

### C) Binding affinity of insulin derivative I to IR-B isoform of human insulin receptor in membranes of murine embryonic fibroblasts

The principle of testing the binding affinity of the insulin derivatives is the same as with the IR-A isoform. However, to test the binding affinities of the insulin derivative to the isoform B of insulin receptor (IR-B) *in vitro,* a cell line of murine embryonic fibroblasts was used, derived from mouse embryonic cells with a deleted (knocked-out) gene for murine IGF-1R (Sell C., Dumenil G. et al., Mol. Cell. Biol. 1994, 14, 3604) and with a high level of expression of the B isoform of human insulin receptor thanks to transfection of cells by this receptor. A procedure by Frasca *et al.* (Frasca F., Pandini G. et al., Mol. Cell. Biol. 1999, 19,3278) was used for the testing.

The cell line of mouse embryo fibroblasts transfected with the human gene for the expression of IR-B (R⁻B) was grown in an incubator at 37 °C in a humidified atmosphere with 5% CO₂ in medium with high glucose (87.6% DMEM medium with 4.5 g/l of glucose, 10% FBS, 2 mmol.l⁻¹ L-Gln, 100 U/ml penicillin, 100 µg/ml streptomycin, 3 µg/ml puromycin) and passaged three times a week. Two days prior to testing, cells were seeded in 24-well plates at a concentration of 3000 cells per well.

The binding affinity was tested in the same way as in the first case, by competition between the tested derivative and ¹²⁵I radiolabeled human insulin - the method by Frasca *et al..* (Frasca F., Pandini G. et al., Mol. Cell. Biol. 1999, 19, 3278). The proliferated cells, approximately 38,000 per well, were washed twice with binding buffer (100 mmol.l⁻¹ HEPES, 100 mmol.l⁻¹ NaCl, 5 mmol.l⁻¹ KCl, 1,3 mmol.l⁻¹ MgSO₄, 1 mmol.l⁻¹ EDTA, 10 mmol.l⁻¹ glucose, 15 mmol.l⁻¹ sodium acetate and 1% BSA, pH 7.6) and the reaction mixture was pipetted into each well in the following order: binding buffer, the corresponding concentration of the tested analogue, and ¹²⁵I*-*insulin with an activity of 43,000 cpm in a total reaction volume of 0.25 ml. The concentration of ¹²⁵I-insulin was 0.043 x 10⁻⁹ mol.l⁻¹. The reaction was incubated for 16 hours at 5 °C with stirring. After completion of the incubation period, the reaction mixture was aspirated, the cells with bound analogues were washed with binding buffer and solubilized with a solution of 0.1 mol.l⁻¹ NaOH. The solution with solubilized cells was transferred to tubes and the radioactivity was measured using a γ-counter (Wizard 1470 Automatic Gamma Counter, Perkin Elmer, Wellesley, USA). Each sample was measured for 60 s. The data were analysed with GraphPad Prism 5.0 using the model of binding to one binding site by non-linear regression, taking into account the potential depletion of the ligand. The value of the dissociation constant (*K*_{d}) of the derivative or of human insulin to the receptor was determined. The concentration of ¹²⁵I-radiolabeled insulin was 0.01 x 10⁻⁹ mol.l⁻¹. The value of 0.3 x 10⁻⁹ mol.l⁻¹ was used as the *K*_{d} of the ¹²⁵I-labelled insulin to the receptor.

The results of the binding studies with insulin derivative **I** and human insulin are shown in Table 1 and in Figure 5.

### D) The binding affinity of insulin derivative I to the human receptor for IGF-1 (IGF-1R) in the membranes of murine embryonic fibroblasts

The principle of testing the binding affinity of the insulin derivatives is the same as with the IR-B isoform. However, to test the binding affinities of the insulin derivative to IGF-1R *in vitro,* a cell line of murine embryonic fibroblasts was used, derived from mouse embryonic cells with a deleted (knocked-out) gene for murine IGF-1R (Sell C., Dumenil G. et al., Mol. Cell. Biol. 1994, 14, 3604) and with a high level of expression of the human receptor for IGF-1 (IGF-1R) thanks to the transfection of cells by this receptor. A procedure by Frasca *et al.* (Frasca F., Pandini G. et al., Mol. Cell. Biol. 1999, 19, 3278) was used for the testing.

The cell line of mouse embryo fibroblasts transfected with the human gene for IGF-1R was grown the same way as the cell line transfected with the IR-B isoform.

Testing binding affinity is done using a competition between the tested derivative and radiolabeled (¹²⁵I) human IGF-1 (Perkin-Elmer, 2497 Ci/mmol). The procedure was the same as with the cells with IR-B with the exception of a few details. The concentration of unlabelled human IGF-1 was determined by measuring absorbance at 280 nm and extinction coefficient (ε) 4560 M⁻¹.cm⁻¹ for human insulin. Approximately 21,000 proliferated cells were used per well and ¹²⁵I-IGF-1 was used with an activity of 44,000 counts per minute, which for the total reaction volume of 0.25 ml corresponds to a ¹²⁵I-IGF-1 concentration of cca. 0.039 x 10⁻⁹ mol.l⁻¹. A value of 0.2 x 10⁻⁹ mol.l⁻¹ was used as the *K*_{d} of ¹²⁵I-labelled IGF-1 to the receptor.

The results of the binding studies with insulin derivative **I** and human insulin are shown in Table 1 and in Figure 6.

**Table 1**

| Values of dissociation constants (*K*_{d}) of human insulin or human IGF-1 and insulin derivative **I** to human insulin receptor in membranes of human IM-9 lymphocytes (IR-A isoform), insulin receptor in membranes of murine fibroblasts transfected either with B isoform (IR-B) of human insulin receptor or human receptor for IGF-1 (IGF-1R). Relative binding affinity of the derivative is expressed in % against human insulin (100%) and is defined as (human insulin *K*_{d} / insulin derivative *K*_{d}) x 100. (n) is the number of measurements. | | | | | | |
|---|---|---|---|---|---|---|
| | *K*_{d} ± S.E. [x 10⁻⁹mol.l⁻¹] (n) | | | Relative binding affinity ± S.E.[%] | | |
| Compound | IR-A | IR-B | IGF-1R | IR-A | IR-B | IGF-1R |
| Human insulin | 0.43±0.02 (5) | 0.67 ± 0.08 (4) | 292 ± 31 (3) | 100 ± 5 | 100 ± 12 | 100 ± 11 |
| Derivative **I** | 0.1 ± 0.02 (3) | 0.13 ± 0.02 (3) | 1327 ± 461 (3) | 226 ± 24 | 515 ± 79 | 22 ± 8 |
| Human IGF-1 | - | - | 0.24 ± 0.05 (5) | - | - | 1217 ± 254 |

Insulin derivative I exhibits an affinity for the IR-A isoform that is double and for IR-B that is up to five times higher than human insulin. The ratio of the relative affinities of the derivative to both isoforms is thus about 2.3 (515%/226%) and is therefore significantly more in favour of the metabolic form of the receptor (IR-B) than that of human insulin. Comparable results were only achieved by Glendorf *et al.* (Glendorf T., Stidsen C. E. et al., Plos One 2011, 6) when they found this ratio for the analogue [HisA8,HisB25,GluB27,des-ThrB30]-insulin 2, but its affinities to both isoforms were significantly lower (67% for IR-A and 140% for IR-B) than those of derivative **I**. A higher ratio of affinities (IR-B%/IR-A%) of 4 was achieved by Glendorf *et al.* for the analogues [HisA8,AsnB25,GluB27,des-ThrB30]-insulin and [HisA8,AsnB25,AspB27,des-ThrB30]-insulin, but only at the expense of lower affinities to the receptors (10-57%). Insulin derivative **I** in its properties therefore possesses not only a preferential affinity for IR-B, but also a very high binding affinity in general (515% relative to human insulin) and thus ranks among the strongest bonds ever found for insulin analogues. Insulin derivative **I** additionally provides another potential advantage over human insulin, and that is its very low affinity receptor for IGF-1 (IGF-1R), which is nearly 5 times lower than the affinity of human insulin.

### E) Determination of NMR structure of insulin derivative I

NMR spectra were acquired at 25°C from a 0.35 mL sample of 1.5 mmol.l⁻¹ derivative **I** in a 20% d₄-acetic acid (pH 1.9) or 25 mmol.l⁻¹ deuterated-Tris buffer (pH 8.0), containing 5% D₂O/95% H₂O on a 600 MHz Bruker Avance spectrometer equipped with a triple-resonance (¹⁵N/¹³C/¹H) cryoprobe. A series of homonuclear spectra were recorded to determine sequence-specific resonance assignments for derivative **I**. In particular, 2D TOCSY with 60 ms mixing time, 2D DQF-COSY and 2D NOESY, which was acquired with an NOE mixing time of 200 ms. Residues involved in forming stable backbone hydrogen bonds were identified by monitoring the rate of backbone amide exchange in 2D TOCSY spectra of derivative **I** dissolved in 20% d₄-acetic acid/80% D₂O. The family of converged structures for derivative I was initially calculated using Cyana 2.1. The combined automated NOE assignment and structure determination protocol was used to automatically assign the NOE cross-peaks identified in 2D NOESY spectrum, and to produce preliminary structures (Herrmann T., Guntert P. et al., J. Mol. Biol 2002, 319, 209). Subsequently, five cycles of simulated annealing combined with redundant dihedral angle constraints (Redac) (Guntert P., Wuthrich K., J. Biomol. NMR 1991, 1, 447) were used to produce a set of converged structures with no significant restraint violations (distance and van der Waals violations < 0.5 Å), which were further refined in explicit solvent using the YASARA software with the YASARA force field (Harjes E., Harjes S. et al., Structure 2006, 14, 881). The 29 structures with the lowest total energy were selected. Analysis of the family of structures obtained was carried out using the programs Molmol (Koradi R., Billeter M. et al., J. Mol. Graph. 1996, 14, 51), Protein structure validation software suite (PSVS) (Bhattacharya A., Tejero R. et al., Proteins 2007, 66, 778) and PyMol (www.pymol.org).

Insulin derivative **I** provided highly similar NMR spectra both in acidic (20% d₃-acetic acid, pH 1.9) or basic (25 mM Tris buffer, pH 8.0) environment indicating that the analogue adopts similar conformation under these conditions. Essentially complete sequence-specific assignment of ¹H NMR resonances has been achieved in 20% acetic acid using the combination of homonuclear NMR experiments. In particular, 98.8% of all proton resonances were assigned with the exception of the poorly resolved H^{ζ} signals of two phenylalanine side-chains (PheB24 and PheB25) and one of the H^{β} protons of CysA11. The ¹H resonance assignments were used for automated assignment of the NOEs identified in 2D NOESY spectrum using Cyana (Herrmann T., Guntert P. et al., J. Mol. Biol. 2002, 319, 209). This yielded unique assignments for 96.8% (886/915) of the NOE peaks observed, providing 764 non-redundant ¹H-¹H distance constraints. The 29 satisfactorily converged structures of derivative **I** (obtained from 100 random starting conformations using 794 NMR-derived structural constraints, including 30 distance restraints for hydrogen bonds (> 16 constraints per residue), and additional 18 constraints for the three disulphide bonds were further refined in explicit water using YASARA (Harjes E., Harjes S. et al., Structure 2006, 14, 881). The distance constraints and structural statistics for obtained structures are in Table 2.

Detailed analysis of the NMR structure (Figure 7) showed that the A-chain of derivative **I** adopted the typical helix-bend-helix conformation type, which is also present in the molecule of human insulin. Likewise, the conformation of the B-chain from PheB1 to GlyB23 is very well conserved. In contrast, the conformations of the human insulin and derivative **I** are fundamentally different in the C-terminus of the B-chain (aminoacids B24-B30). The covalent triazole arm between positions B26 and B29 of derivative **I** directs the peptide chain of the derivative (position B25-B30) into a bent conformation, with the triazole linker directed approximately to the place where the human insulin has a side chain TyrB26. However, the entire peptide chain of derivative **I** is in positions B25-B30 further away from the central part of the molecule. Incorporation of the triazole arm between positions B26 and B29 brought greater looseness and flexibility to derivative **I** in this part of the molecule, which is the cause of its higher affinity for both isoforms of the insulin receptor. This derivative of insulin can switch to the active conformation more easily than human insulin upon binding to the receptor, which, as we believe, is characterized by a B26-bend in this part of the B-chain or by similar bending (Jirá 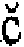 ek J., 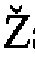 áková L. *et al*., *Proc. Natl. Acad. Sci. U.S.A.* **2010,** 107, 1966; 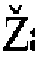 áková L., Kletviková E. *et al*., *J. Biol. Chem.* **2013**, 288, 10230). Loosening the structure of the derivative at the C-terminus of the B-chain also results in a disturbance of its dimerization region, which decreases its ability to form dimers, and thus allows a faster onset of its action after application *in vivo.*

**Table 2. NMR constraints and structural statistics for derivative I.**

| | |
|---|---|
| *Non-redundant distance constrains* | 794 |
| Intra-residue NOEs [i = j] | 219 |
| Sequential NOEs [\|i - j\| = 1] | 215 |
| Medium-range NOEs [1 < \|i - j\| < 5] | 162 |
| Long-range NOEs [\|i - j \| ≥ 5] | 165 |
| Experimental hydrogen bond constraints | 30 |
| Constrains per residue | 16.2 |
| Total structures calculated | 100 |
| Number of structures used | 29 |
| *Average number of distance constraints violations* | |
| 0.1 x 10⁻¹⁰ m to 0.2 x 10⁻¹⁰ m | 5.14 |
| 0.2 x 10⁻¹⁰ m to 0.5 Å x 10⁻¹⁰ m | 2.45 |
| > 0.5 x 10⁻¹⁰ m | 0 |
| r.m.s. of distance violation / constraint (x 10⁻¹⁰ m) | 0.03 |
| Maximum distance violation (x 10⁻¹⁰ m) | 0.49 |
| *Ramachandran plot summary from Procheck* | |
| Residues within the most favoured region | 90.4% |
| Residues within the additionally allowed region | 9.4% |
| Residues within generously allowed region | 0.1% |
| Residues within the disallowed region | 0.1% |
| *r.m.s.d. to the mean structure (chain A₁₋₂₁ and chain B₁₋₂₃)* | |
| Backbone heavy atom (x 10⁻¹⁰ m) | 1.1 ± 0.3 |
| All heavy atom (x 10⁻¹⁰ m) | 2.0 ± 0.4 |

### Industrial applicability

The insulin derivative of formula **I** according to this invention is suitable as an active ingredient in pharmaceutical compositions for treating or preventing diabetes and conditions characterized by elevated levels of blood glucose, *i.e.* for the reduction of blood glucose concentration. Due to its high affinity for the insulin receptor, in particular to metabolic isoform B, and very low affinity for the receptor for IGF-1 and reduced ability to dimerize, the derivative has a significant potential for use as a preparation with a rapid onset of action and a reduced risk of developing cancer.

## Claims

1. Insulin derivative with cyclic structure in the C-terminus of B-chain having formula **I** 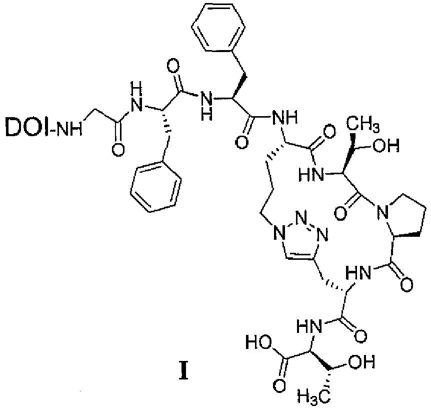 where DOI is des(B23-B30)octapeptide-insulin,
and its pharmaceutically acceptable salts and solvates.

2. Insulin derivative of the formula **I** according to claim 1, or its pharmaceutically acceptable salt or solvate, for use as a medicament.

3. Insulin derivative with formula **I** according to claim 1, or its pharmaceutically acceptable salt or solvate, for use in a method of treatment or prevention of a condition involving hyperglycemia or for diabetes treatment.

4. Pharmaceutical composition, **characterized in that** it contains the insulin derivative of formula **I** according to claim 1, and/or at least one pharmaceutically acceptable salt or solvate thereof.

5. Pharmaceutical composition according to claim 4, further containing at least one pharmaceutically acceptable carrier, excipient and/or diluent.

6. Pharmaceutical composition according to claim 4 or 5, for use in a method of treatment or prevention of a condition involving hyperglycemia or for diabetes treatment.

## Patentansprüche

1. Insulin-Derivat mit cyclischer Struktur am C-Terminus der B-Kette der Formel **I** 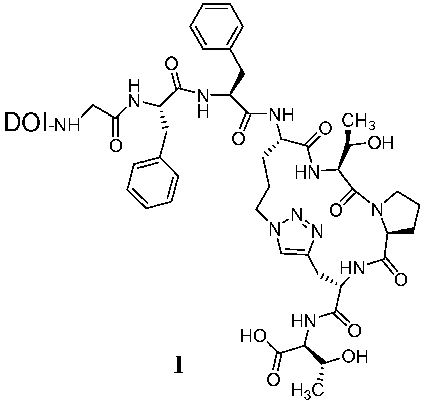 worin DOI ein Des(B23-B30)octapeptidinsulin ist,
und ihre pharmazeutisch annehmbaren Salze und Solvate.

2. Insulin-Derivat der Formel I nach Anspruch 1 oder sein pharmazeutisch annehmbares Salz oder Solvat zur Verwendung als Medikament.

3. Insulin-Derivat der Formel I nach Anspruch 1 oder sein pharmazeutisch annehmbares Salz oder Solvat zur Verwendung in einem Verfahren zur Behandlung oder Prävention einer Krankheit die Hyperglykämie involviert oder zur Behandlung von Diabetes.

4. Pharmazeutische Zusammensetzung, **dadurch gekennzeichnet, dass** sie das Insulin-Derivat der Formel I nach Anspruch 1 und/oder mindestens ein pharmazeutisch annehmbares Salz oder Solvat davon enthält.

5. Pharmazeutische Zusammensetzung nach Anspruch 4, weiterhin enthaltend mindestens einen pharmazeutisch annehmbaren Träger, Hilfstoff und/oder Verdünnungsmittel.

6. Pharmazeutische Zusammensetzung nach Anspruch 4 oder 5 zur Verwendung in einem Verfahren zur Behandlung oder Prävention einer Krankheit die Hyperglykämie involviert oder zur Behandlung von Diabetes.

## Revendications

1. Dérivé d'insuline ayant une structure cyclique dans l'extrémité C-terminale de la chaîne B ayant la formule I 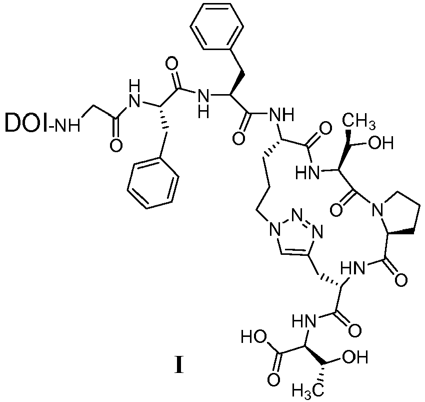 dans lequel DOI est des(B23-B30)octapeptide-insuline,
et ses sels et solvates pharmaceutiquement acceptables.

2. Dérivé d'insuline de formule I selon la revendication 1, ou son sel ou solvate pharmaceutiquement acceptable, pour utilisation comme médicament.

3. Dérivé d'insuline de formule I selon la revendication 1, ou son sel ou solvate pharmaceutiquement acceptable, pour utilisation dans une méthode de traitement ou de prévention d'un état impliquant une hyperglycémie ou pour un traitement du diabète.

4. Composition pharmaceutique, **caractérisée en ce qu'**elle contient le dérivé d'insuline de formule I selon la revendication 1, et/ou au moins un de ses sels ou solvates pharmaceutiquement acceptables.

5. Composition pharmaceutique selon la revendication 4, contenant en outre au moins un véhicule, excipient et/ou diluant pharmaceutiquement acceptable.

6. Composition pharmaceutique selon la revendication 4 ou 5, pour utilisation dans une méthode de traitement ou de prévention d'un état impliquant une hyperglycémie ou pour un traitement du diabète.
